# EUROPÄISCHE PATENTSCHRIFT

(11) **EP 3 322 027 B1**
(45) Veröffentlichungstag und Bekanntmachung des Hinweises auf die Patenterteilung: **24.07.2019**
(21) Anmeldenummer: 17174216.6
(22) Anmeldetag: 02.06.2017
(51) Int. Cl.: G01R 33/36, H04B 5/00, G01R 33/341, H05K 1/16

(54) **NAHFELD KOPPLER ZUR ULTRA BREIT BAND SIGNALÜBERTRAGUNG.**
NEAR FIELD COUPLER FOR TRANSMITTING UWB SIGNALS.
COUPLEUR À CHAMP PROCHE DESTINÉ À LA TRANSMISSION DE SIGNAUX ULTRA LARGE BANDE.

(43) Veröffentlichungstag der Anmeldung: 16.05.2018
(73) Patentinhaber: Siemens Healthcare GmbH, 91052 Erlangen (DE)
(72) Erfinder: Fackelmeier, Andreas, 91177 Thalmässing (DE)

(56) Entgegenhaltungen:
- WO-A1-2009/069097
- US-A- 4 527 163
- US-A1- 2012 326 520
- US-A1- 2016 020 508
- Debatosh Guha ET AL: "Microstrip and Printed Antennas - New Trends, Techniques and Applications" In: "Microstrip and Printed Antennas - New Trends, Techniques and Applications", 1. Januar 2011 (2011-01-01), John Wiley & Sons Inc, New York, NY, US, XP055337090, ISBN: 978-0-470-68192-3 Seiten i-481, * printed UWB antenna; Absätze [01.8], [0010], [08.6] * * near field calculations; Absätze [02.4], [08.2] * * UHF Passive RFID Tag Antennas; Meander dipole; Absatz [09.3] *

## Beschreibung

Die Erfindung betrifft einen Koppler zur Signalübertragung, umfassend ein erstes Kopplerelement mit einem ersten Übertragungselement und ein zweites Kopplerelement mit einem zweiten Übertragungselement, wobei an einem ersten Ende des ersten Übertragungselements ein erster Signalkontakt zur Verbindung mit einem Signalleiter angeordnet ist, wobei an einem ersten Ende des zweiten Übertragungselements ein zweiter Signalkontakt zur Verbindung mit einem Signalleiter angeordnet ist, wobei das erste Übertragungselement dazu eingerichtet ist, im Betrieb ein am ersten Signalkontakt eingespeistes Signal an das zweite Übertragungselement zu übertragen, und wobei das zweite Übertragungselement dazu eingerichtet ist, im Betrieb das Signal vom ersten Übertragungselement zu empfangen und über den zweiten Signalkontakt auszugeben.

In vielen unterschiedlichen Anwendungen tritt die Anforderung auf, bei einer Datenerzeugung während eines technischen Vorgangs die erzeugten Daten mit einer möglichst hohen Datenrate von einer datenerzeugenden Komponente eines Gerätes zu einer datenverarbeitenden Komponente des Geräts zu übertragen, welche von der datenerzeugenden Komponente räumlich getrennt ist. Diese Anforderung tritt unabhängig von der Art der Datenerzeugung sowie unabhängig von der Art und auch dem Zweck ihrer Weiterverarbeitung in vielen technischen Gebieten auf. In vielen Fällen wird dabei die Implementation der Datenübertragung komplett auf den übergeordneten technischen Prozess abgestimmt, das heißt, es wird eine eigenständige technische Lösung zur Datenübertragung entwickelt, insbesondere, wenn besonders hohe Datenraten zu bewältigen sind, und eine Vorverarbeitung der Daten, welche für eine Einbettung in standardisierte Übertragungsprotokolle meist erforderlich ist, bei der Datenerzeugung nicht erwünscht oder schlicht nicht machbar ist.

Gerade in der bildgebenden Medizintechnik, wo in sehr kurzer Zeit vergleichsweise große Datenmengen an Bilddaten generiert werden, welche von oftmals beweglichen Geräteteilen an eine die entsprechenden Rekonstruktionsalgorithmen durchführende Recheneinheit zu übertragen sind, ist dies ein häufiges Szenario. Der Trend zu immer höheren Bildauflösungen bei jedem erzeugten Einzelbild aufgrund der wachsenden physikalischen Möglichkeiten der Bildgebung sowie zu einer möglichst immer kürzeren Abfolge einzelner Aufnahmen, um eine Verfälschung durch Körperbewegungen zu vermeiden, verschärfen die Herausforderungen bei der besagten Datenübertragung. Bei einer Magnetresonanztomographie (MRT) sind beispielsweise proprietäre Datensignale von einer Lokalspule zu einer Patientenliege zu übertragen. Ein weiteres Beispiel für einen Übertragungsprozess findet sich bei den in einem Schallkopf erzeugten Empfangssignalen zu einem Ultraschallgerät.

Für die genannten Anwendungen werden infolge der großen zu übertragenden Datenmengen oftmals kostenintensive, großvolumige Stecker und entsprechend dicke Kabel verwendet. Derartige Stecker und Kabel bringen infolge der ungünstigen Handhabbarkeit und auch des hohen Gewichts in Betrieb mit bildgebenden medizinischen Geräten Nachteile mit sich. Eine Lokalspule eines MRT muss zum Beispiel wegen des hohen Gewichts der zu verwendenden Übertragungskabel extra befestigt werden, damit sie durch das Kabel nicht verschoben wird. Die verwendeten Stecker weisen zudem oftmals eine Vielzahl von Kontakten auf, da sehr viele Einzelkanäle übertragen werden müssen, wobei diese Kontakte oftmals schwierig zu reinigen sind, und zudem oftmals auch keine hohe Steckzyklen-Fähigkeit aufweisen. Hierbei ist insbesondere zu berücksichtigen, dass selbst beim Vorsehen von Redundanzkanälen im Übertragungsprotokoll ein Stecker bei einem Defekt von wenigen Kontakten die Datenübertragung möglicherweise nicht mehr gewährleisten kann.

Die genannten Anforderungen sind hierbei nicht nur auf Stecker als Übertragungsmittel beschränkt, sondern lassen sich auf nahezu jede Art von Koppler zur Signalübertragung bei hohen Datenraten ausdehnen.

In der US 2012 / 0 326 520 A1 ist ein zweiteiliger Koppler genannt, in welchem die für die Signalübertragung vorgesehenen Übertragungselemente jeweils durch Streifenleiter gebildet werden, wobei jeder Streifenleiter jeweils in zwei Ebenen eine komplexe, dreidimensionale Windungsstruktur ausbildet und dabei über Stufen zwischen den beiden Ebenen wechselt. Die Draufsicht eines jeden Streifenleiters bildet hierbei ein mäanderförmiges Muster. Die Übertragungselemente zur Signalübertragung sind jeweils von Spulen zur induktiven Übertragung von Energie zwischen den beiden Teilen des Kopplers umgeben.
US2016020508 definiert Spiralförmige Antennen für "Near Field Communication".

Der Erfindung liegt daher die Aufgabe zugrunde, einen Koppler zur Signalübertragung anzugeben, welcher eine möglichst hohe Datenrate aufweist, dessen Koppelverhalten möglichst robust gegen mechanische Toleranzen ist, und welcher eine möglichst kompakte Bauweise erlaubt.

Die genannte Aufgabe wird erfindungsgemäß gelöst durch einen Koppler zur kontaktlosen Signalübertragung, umfassend ein erstes Kopplerelement mit einem ersten Übertragungselement und ein zweites Kopplerelement mit einem zweiten Übertragungselement, wobei an einem ersten Ende des ersten Übertragungselements ein erster Signalkontakt zur Verbindung mit einem Signalleiter angeordnet ist, wobei an einem ersten Ende des zweiten Übertragungselements ein zweiter Signalkontakt zur Verbindung mit einem Signalleiter angeordnet ist, wobei das erste Übertragungselement zwischen dem ersten Ende und einem zweiten Ende eine Mehrzahl an ersten U-förmigen Kurven ausbildet und dabei eine Spirale ausbildet oder um eine Mantelfläche eines Zylinders gewickelt ist, wobei das zweite Übertragungselement zwischen dem ersten Ende und einem zweiten Ende eine Mehrzahl an zweiten U-förmigen Kurven ausbildet und dabei eine Spirale ausbildet oder um eine Mantelfläche eines Zylinders gewickelt ist, wobei das erste Übertragungselement dazu eingerichtet ist, im Betrieb ein am ersten Signalkontakt eingespeistes Signal mittels Richtkopplung an das zweite Übertragungselement zu übertragen, und wobei das zweite Übertragungselement dazu eingerichtet ist, im Betrieb das Signal vom ersten Übertragungselement zu empfangen und über den zweiten Signalkontakt auszugeben. Vorteilhafte und teils für sich gesehen erfinderische Ausgestaltungen sind in den Unteransprüchen und in der nachfolgenden Beschreibung dargelegt.

Insbesondere kann der Koppler für eine bidirektionale Signalübertragung eingerichtet sein, sodass das zweite Übertragungselement dazu eingerichtet ist, im Betrieb ein am zweiten Signalkontakt eingespeistes Signal mittels Richtkopplung an das erste Übertragungselement zu übertragen, und das erste Übertragungselement dazu eingerichtet ist, im Betrieb das Signal vom zweiten Übertragungselement zu empfangen und über den ersten Signalkontakt auszugeben.

Unter einem Koppler ist hierbei generell eine zweielementige Vorrichtung umfasst, welche dazu eingerichtet ist, im Betrieb ein Signal zwischen den beiden Elementen zu übertragen, wobei die beiden Elemente reversibel voneinander trennbar ausgestaltet sind. Bevorzugt sind das erste Übertragungselement und das zweite Übertragungselement jeweils derart ausgebildet, dass sich lokal eine Vorzugsrichtung definieren lässt, so dass eine Richtkopplung lokal bezüglich dieser Vorzugsrichtung erfolgt, welche innerhalb des jeweiligen Übertragungselements auch den Signalweg bestimmt. Die lokalen Vorzugsrichtungen erstrecken sich dann jeweils vom am ersten Ende des betreffenden Übertragungselements angeordneten Signalkontakt entlang des Übertragungselements bis zum zweiten Ende hin.

Unter einer U-förmigen Kurve ist hier generell jede Krümmung des betreffenden Übertragungselementes um 180° umfasst, also insbesondere auch eine Kurve ohne jegliche geraden einlaufenden oder auslaufenden Abschnitte zur eigentlichen Krümmung hin oder von dieser weg. Ein Ausbilden einer Mehrzahl an U-förmigen Kurven durch eines der beiden Übertragungselemente zwischen seinem ersten Ende und seinem zweiten Ende ist hierbei insbesondere so zu verstehen, dass durch die Gesamtheit der lokalen Vorzugsrichtungen des betreffenden Übertragungselements zwischen dem ersten Ende und dem zweiten Ende ein Linienverlauf gegeben ist, wobei die Linien beispielsweise gegeben sein können durch die lokalen Begrenzungen des Übertragungselementes in einer zur Vorzugsrichtung lokal senkrechten Raumrichtung, und dass der Linienverlauf die entsprechenden U-förmigen Kurven aufweist.

Die Mehrzahl an U-förmigen Kurven kann hierbei einerseits stets dieselbe Krümmungsrichtung aufweisen, wie es beispielsweise bei einer Spirale der Fall ist, oder andererseits die Krümmungsrichtung auch alternieren, wodurch ein mäanderförmiger Linienverlauf ausgebildet wird. Insbesondere können dabei einzelne U-förmige Kurven voneinander durch gerade Abschnitte im Linienverlauf des jeweiligen Übertragungselements voneinander getrennt sein und insbesondere können auch einzelne U-förmige Kurven zwischen einem Eingangspunkt und einem Ausgangspunkt der jeweiligen Kurve eine Anzahl an geraden Abschnitten ohne eine Krümmung der Vorzugsrichtung bzw. der Linienführung aufweisen, so dass sich die gesamte Krümmung zwischen dem Eingangspunkt und dem Ausgangspunkt zu 180° addiert. Ebenso ist es möglich, dass ein Übertragungselement in seinem Linienverlauf keinerlei gerade Abschnitte aufweist, sondern durch eine kontinuierliche Aneinanderreihung von U-förmigen Kurven gebildet wird, welche ihrerseits jeweils eine stetige Krümmung aufweisen. Dies ist beispielsweise bei einem als eine Spirale ausgebildeten Übertragungselement, oder bei einem entlang eines Kegelmantels aufgewickelten Übertragungselement, oder bei einem um einen Zylindermantel aufgewickelten Übertragungselement der Fall. Besonders bevorzugt bilden dabei die zweiten U-förmigen Kurven des zweiten Übertragungselementes in ihrer räumlichen Ausrichtung die ersten U-förmigen Kurven des ersten Übertragungselementes nach.

Unter einer Richtkopplung ist hierbei insbesondere das gegenseitige Übersprechen zweier in unmittelbarer räumlicher Nähe zueinander befindlicher elektrischer Leiter, welche jedoch nicht miteinander in leitendem Kontakt stehen, zu verstehen. Durch die Verwendung einer solchen Richtkopplung wird im erfindungsgemäßen Koppler erreicht, auf einen physischen Kontakt zwischen dem ersten Übertragungselement und dem zweiten Übertragungselement verzichten zu können. Bei einem solchen physischen Kontakt sind oftmals je nach Art der Datenübertragung und gegebenenfalls des Übertragungsprotokolls eine Vielzahl an einzelnen Übertragungskanälen am ersten Übertragungselement und am zweiten Übertragungselement eines Kopplers nach dem Stand der Technik gesondert zu kontaktieren. Dies führt entweder zu einer unvorteilhaften Dimensionierung des entsprechenden Kopplers oder zu besonders kleinen Kontaktstellen, welche bei einem häufigen Trennen der beiden Kopplerelemente voneinander infolge des physischen Kontakts gegebenenfalls leicht verschleißen könnten.

Richtkoppler, wie sie in der Hochfrequenztechnik Anwendung finden, nutzen hierbei als Übertragungselemente meist zwei planare, streifenförmige Leiter, wobei die für die Signalübertragung wirksame Strecke bei beiden Leitern üblicherweise in der Größenordnung von einem Viertel der Wellenlänge des zu übertragenden Signals liegt. Auch wenn hierbei durch einige bauliche Ausgestaltungen, wie zum Beispiel für den Abstand der beiden Leiter während der Übertragung oder die Verwendung von dielektrischen Substraten zwischen den beiden Leitern einiger Gestaltungsspielraum besteht, wird hierdurch die prinzipielle Größenordnung der Übertragungsstrecke nicht berührt.

Für viele Anwendungen, insbesondere solche, bei welchen die beiden Kopplerelemente häufiger voneinander zu trennen sind, also unter anderem auch im Bereich im bildgebenden Medizintechnik, sind jedoch planare Streifenleiter als Übertragungselemente infolge der aufwändigen Positionierung zueinander, infolge der hohen Empfindlichkeit gegenüber mechanischen Toleranzen, und infolge der relativen Länge der Übertragungsstrecke unpraktisch. Dieses Problem bei der Verwendung von Richtkopplern wird nun durch die Erfindung dadurch gelöst, dass das erste Übertragungselement und das zweite Übertragungselement durch über die jeweiligen U-förmigen Kurven "aufgewickelt" werden, wodurch einerseits der Platzbedarf erheblich reduziert werden kann, und andererseits die Positionierung der beiden Übertragungselemente relativ zueinander infolge der deutlich verbesserten Robustheit gegenüber Abweichungen deutlich weniger aufwändig gestaltet werden muss. Dadurch, dass nämlich die beiden Übertragungselemente durch die U-förmigen Kurven jeweils eine flächenartige Ausdehnung gewinnen, sind nun für die Ausnutzung des Richtkoppler-Effekts nicht mehr zwei streifenförmige Leiter relativ zueinander zu positionieren, sondern im Wesentlichen zwei Flächen aneinander auszurichten, was sich oftmals erheblich einfacher gestaltet. Die Befestigung des ersten Kopplerelements mit dem zweiten Kopplerelement für den Betrieb des Kopplers kann hierbei zudem komplett getrennt von der Signalübertragung konzipiert werden, und gegebenenfalls bei Verschleiß auch gesondert von dieser ausgetauscht werden, was die Lebensdauer des Kopplers zusätzlich erhöht.

Erfindungsgemäß liegen dabei das erste Übertragungselement und das zweite Übertragungselement jeweils in einer Ebene, oder das erste Übertragungselement und das zweite Übertragungselement verlaufen entlang von Rotationsflächen, welche zueinander komplementär sind. Eine Rotationsfläche ist hierbei eine Fläche, die durch die Rotation einer ebenen Kurve um eine in derselben Ebene liegenden Rotationsachse entsteht. Insbesondere ist hierbei also auch eine Zylinderoberfläche umfasst. Vorteilhafterweise verläuft hierbei die Rotationsfläche in einer Richtung der Rotationsachse unter einer monotonen Vergrößerung des Abstands von der Rotationsachse. Durch den beschriebenen Verlauf jeweils in einer Ebene oder entlang von komplementären Rotationsflächen lassen sich das erste Übertragungselement und das zweite Übertragungselement besonders einfach aneinander ausrichten, wobei durch den Verlauf entlang komplementärer Rotationsflächen zusätzlich gewährleistet werden kann, dass das erste Übertragungselement und das zweite Übertragungselement im Betrieb wenigstens abschnittsweise einen geringen Abstand zueinander einnehmen, was für die kontaktlose Übertragung vorteilhaft ist.

Durch die intrinsische Rotationssymmetrie der beiden Ebenen bzw. durch die Rotationssymmetrie der beiden zueinander komplementären Rotationsflächen ist es zudem möglich, das erste Übertragungselement und das zweite Übertragungselement jeweils so auszugestalten, dass Rotationen bezüglich einer gemeinsamen Symmetrieachse nur eine begrenzte Wirkung auf eine Kopplungsstärke der Richtkopplung entfalten. Dies ist besonders vorteilhaft für die Positionierung des ersten Kopplerelements relativ zum zweiten Kopplerelement.

Zweckmäßigerweise ist dabei das erste Kopplerelement rotationssymmetrisch ausgebildet, und das zweite Kopplerelement komplementär zum ersten Kopplerelement ausgebildet. Bevorzugt wird hierbei eine rotationssymmetrische Form des ersten Kopplerelements durch eine entsprechende Fläche, in der das erste Übertragungselement verläuft, vorgegeben. Durch die Rotationssymmetrie und die komplementäre Ausgestaltung lassen sich das erste Kopplerelement und das zweite Kopplerelement mechanisch besonders einfach miteinander verbinden.

Erfindungsgemäß bildet das erste Übertragungselement zwischen dem ersten Ende und dem zweiten Ende eine Spirale aus. Insbesondere verläuft hierbei die Spirale in einer Ebene oder entlang eines Kegelmantels. Erfindungsgemäß bildet das zweite Übertragungselement zwischen seinem ersten Ende und seinem zweiten Ende eine Spirale aus, welche besonders bevorzugt der Spirale des ersten Übertragungselements entspricht. Gerade bei einer derartigen Ausgestaltung kann auch noch bei einer erheblichen Verdrehung des ersten Übertragungselements gegen das zweite Übertragungselement bezüglich des gemeinsamen Spiralenzentrums ein hoher Grad an räumlichem Überlapp und somit eine hohe Kopplungsstärke für die Richtkopplung gewährleistet werden.

Als Alternative hierzu ist im Rahmen der Erfindung vorgesehen, dass das Übertragungselement um eine Mantelfläche eines Zylinders gewickelt ist. Erfindungsgemäß ist hierbei auch das zweite Übertragungselement auf eine dem ersten Übertragungselement entsprechenden Weise um eine Mantelfläche des Zylinders gewickelt. Durch eine derartige Ausgestaltung lassen sich die beiden Übertragungselemente als koaxiale Zylinder formen, wobei für den Betrieb des Kopplers ein zylinderförmiges Kopplerelement in eine entsprechende zylinderförmige Hülse, welche durch das andere Kopplerelement gebildet wird, eingeführt wird. Bei einer Trennung der beiden Kopplerelemente voneinander ist so insbesondere das Übertragungselement in der zylinderförmigen Hülse besonders gut vor externen Einflüssen geschützt.

Als weiter vorteilhaft erweist es sich, wenn das erste Übertragungselement und/oder das zweite Übertragungselement wenigstens abschnittsweise lokal flächenartig ausgebildet sind. Insbesondere ist hierunter eine streifenartige Ausgestaltung umfasst. Unter lokal flächenartig ist hierbei zu verstehen, dass senkrecht zur lokalen Vorzugsrichtung im betreffenden Übertragungselement lokal in einer Richtung eine Abplattung, in der anderen Raumrichtung senkrecht zur lokalen Vorzugsrichtung jedoch eine Verbreiterung erfolgt. Insbesondere sind dabei sowohl die Abplattung als auch die Verbreiterung bezogen auf die Gesamtquerschnittsfläche erheblich. Bevorzugt ist hierdurch lokal ein im Wesentlichen rechteckiger oder elliptischer Querschnitt gegeben, dessen beide Raumrichtungen sich um wenigstens eine Größenordnung voneinander unterscheiden. Durch eine wenigstens abschnittsweise lokale Flächenartigkeit des ersten Übertragungselements und/oder des zweiten Übertragungselements lässt sich die Kopplungsstärke der Richtkopplung verbessern. Insbesondere sind das erste Übertragungselement und das zweite Übertragungselement jeweils entlang ihrer vollständigen, durch die entsprechende Linienführung definierten Länge zwischen dem jeweils ersten und zweiten Ende lokal flächenartig ausgebildet.

Bevorzugt ist das erste Übertragungselement auf einem dielektrischen Substrat aufgebracht, welches eine Oberfläche des ersten Kopplerelements bildet. Besonders bevorzugt ist auch das zweite Übertragungselement auf einem dielektrischen Substrat aufgebracht, welches eine Oberfläche des zweiten Kopplerelements bildet. Als ein dielektrisches Substrat kann hierbei insbesondere FR4 verwendet sein. Insbesondere kann das dielektrische Substrat durch einen elektrischen Isolator gegeben sein. Die Verwendung eines derartigen dielektrischen Substrats, mit welchem das erste Übertragungselement bzw. das zweite Übertragungselement zur Oberfläche des jeweiligen Kopplerelements hin überzogen ist, erlaubt es, für den Betrieb des Kopplers den Abstand zwischen dem ersten Übertragungselement und dem zweiten Übertragungselement auf besonders einfache Weise zu kontrollieren. Die beiden Kopplerelemente werden - bevorzugt formschlüssig - in Kontakt gebracht, sodass an den entsprechenden Oberflächen das dielektrische Substrat für die korrekte Beabstandung der beiden Übertragungselemente sorgt. Dies ist insbesondere vor dem Hintergrund bedeutsam, dass bei Richtkopplern der Abstand der Übertragungselemente zueinander einen maßgeblichen Einfluss auf die Kopplungsstärke und auch auf den Frequenzgang hat.

Als weiter vorteilhaft erweist es sich, wenn im ersten Kopplerelement eine erste Spule zur induktiven Energieübertragung angeordnet ist, und im zweiten Kopplerelement eine zweite Spule zur induktiven Energieübertragung angeordnet ist, wobei die erste Spule und die zweite Spule dazu eingerichtet sind, im Betrieb des Kopplers Energie vom ersten Kopplerelement zum zweiten Kopplerelement und/oder vom zweiten Kopplerelement zum ersten Kopplerelement zu übertragen. Dies erlaubt es, den Koppler nicht nur zur Signalübertragung, sondern auch zur Energieübertragung zu nutzen, wodurch in der übergeordneten Anwendung auf eine gesonderte Energieübertragungsstrecke verzichtet werden kann. Hierbei wird auf vorteilhafte Weise insbesondere der Umstand ausgenutzt, dass eine induktive Energieübertragung nicht in nennenswerter Weise mit der im Koppler zur Signalübertragung verwendeten Richtkopplung interferiert.

Zweckmäßigerweise weisen das erste Kopplerelement und das zweite Kopplerelement jeweils einen elektromagnetischen Absorber auf. Dies ist insbesondere dann von Vorteil, wenn im ersten Kopplerelement und im zweiten Kopplerelement jeweils wenigstens eine Spule zur induktiven Energieübertragung angeordnet ist, sodass im Betrieb des Kopplers eine über die betreffenden Spulen erfolgende induktive Energieübertragung durch die jeweiligen elektromagnetischen Absorber abgeschirmt werden kann, und somit nicht mit der übergeordneten Anwendung interferiert.

Bevorzugt ist der Koppler für eine Übertragung von Signalen in einem Frequenzbereich von 200 MHz bis 4 GHz, besonders bevorzugt in einem Frequenzbereich von 300 MHz bis 2,5 GHz eingerichtet. Für eine Signalübertragung mittels Richtkopplung im genannten Frequenzbereich sind wirksame Kopplungslängen erforderlich, welche vom Zentimeter- bis in den Dezimeterbereich reichen können. Bei derartigen wirksamen Kopplungslängen ist die vorgeschlagene "Aufwicklung" des ersten Übertragungselements und des zweiten Übertragungselements mittels entsprechender U-förmiger Kurven besonders vorteilhaft für die Abmessungen des Kopplers.

Die Erfindung nennt weiter einen Magnetresonanztomographen mit einer Lokalspule und einem vorbeschriebenen Koppler, wobei das erste Kopplerelement des Kopplers an der Lokalspule angeordnet ist. Die für den Koppler und seine Weiterbildungen angegebenen Vorteile können hierbei sinngemäß auf den Magnetresonanztomographen übertragen werden.

Nachfolgend wird ein Ausführungsbeispiel der Erfindung anhand einer Zeichnung näher erläutert. Hierbei zeigen jeweils schematisch:
- FIG 1: in einer Schnittdarstellung eine Lokalspule eines MRT mit einem Koppler,
- FIG 2: in einer perspektivischen Darstellung zwei eben spiralförmige Übertragungselemente eines Kopplers nach FIG 1,
- FIG 3: in einer Seitenansicht zwei spiralförmige Übertragungselemente eines Kopplers, welche jeweils entlang von Kegelmänteln verlaufen, und
- FIG 4: in einer Seitenansicht zwei Übertragungselemente eines Kopplers, welche jeweils entlang von Zylindermänteln verlaufen.

Einander entsprechende Teile und Größen sind in allen Figuren jeweils mit gleichen Bezugszeichen versehen.

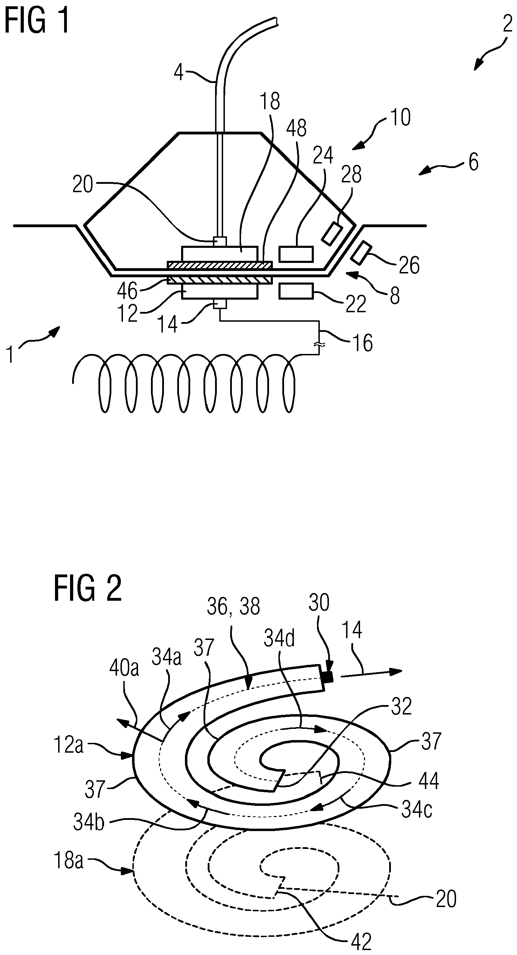

In FIG 1 ist schematisch in einer Schnittdarstellung eine Lokalspule 1 eines MRT 2 gezeigt, zu welcher mittels eines Kabels 4 von einer nicht dargestellten Patientenliege des MRT 2 durch einen Koppler 6 eine reversibel trennbare Datenverbindung herstellbar ist. Der Koppler 6 umfasst ein erstes Kopplerelement 8, welches an der Lokalspule 1 angeordnet ist, und ein zweites Kopplerelement 10, welches am Ende des Kabels 4 angeordnet ist. Das erste Kopplerelement 8 weist ein noch zu beschreibendes erstes Übertragungselement 12 mit einem ersten Signalkontakt 14 auf, welcher mit einer Signalleitung 16 der Lokalspule 1 kontaktiert ist. Das zweite Kopplerelement 10 weist ein noch zu beschreibendes zweites Übertragungselement 18 mit einem zweiten Signalkontakt 20 auf, an welchem das Ende des Kabels 4 kontaktiert ist. Weiter weisen das erste Kopplerelement 8 und das zweite Kopplerelement 10 jeweils eine erste Spule 22 bzw. eine zweite Spule 24 für eine Energieversorgung der Lokalspule 1 mittels induktiver Energieübertragung sowie elektromagnetische Absorber 26, 28 auf, damit die induktive Energieübertragung möglichst wenig mit dem Gesamtbetrieb des MRT 2 interferiert. Im Betrieb des MRT 2 werden nun medizinische Bilddaten, welche in der Lokalspule 1 erzeugt werden, vom ersten Übertragungselement 12 des ersten Kopplerelements 8 an das zweite Übertragungselement 18 des zweiten Kopplerelements 10 mittels Richtkopplung übertragen, und die Lokalspule zudem über die Spulen 22, 24 mit Leistung versorgt.

In FIG 2 sind in einer perspektivischen Darstellung das erste Übertragungselement 12a und das zweite Übertragungselement 18a des Kopplers 6 nach FIG 1 gezeigt. An einem ersten Ende 30 des ersten Übertragungselements 12a ist der erste Signalkontakt 14 angeordnet. Das erste Übertragungselement 12a verläuft bis zu seinem freien zweiten Ende 32 hin spiralförmig. Insbesondere lässt sich hierbei jeweils lokal eine Vorzugsrichtung 34a-d definieren, welche durch den lokalen Signalverlauf im ersten Übertragungselement 12a bestimmt wird. Durch die Gesamtheit 36 der lokalen Vorzugsrichtungen 34a-d ist somit ein Linienverlauf gegeben, welcher vorliegend eine Mehrzahl an aneinandergereihten U-förmigen Kurven 37 gleicher Krümmungsrichtung und somit eine Spirale 38 bildet. In einer Richtung 40a senkrecht zur lokalen Vorzugsrichtung 34a weist das erste Übertragungselement 12 eine merkliche räumliche Ausdehnung auf, wobei in der zur Richtung 40a und zur lokalen Vorzugsrichtung 34a senkrechten Raumrichtung (nicht dargestellt) das erste Übertragungselement 12a keine nennenswerte Ausdehnung aufweist, und somit eine lokal flächenartige, streifenförmige Struktur ausbildet.

Das zweite Übertragungselement 18a bildet von seinem ersten Ende 42 zu seinem freien zweiten Ende 44 hin die Spiralform des ersten Übertragungselements 12a nach. Am ersten Ende 42 ist der zweite Signalkontakt 20 angeordnet, an welchem das Kabel 4 kontaktiert ist. Sowohl das erste Übertragungselement 12a als auch das zweite Übertragungselement 18a sind auf einem dielektrischen Substrat 46, 48 aufgebracht, welches jeweils die Oberfläche des ersten bzw. zweiten Kopplerelements 8, 10 im gemeinsamen Kontaktbereich bildet.

Durch die global spiralförmige, lokal jedoch flächen- bzw. streifenartige Ausgestaltung des ersten und zweiten Übertragungselements 12a, 18a kann einerseits für die Richtkopplung eine deutlich höhere Kopplungsstärke erreicht werden als bei global linienförmigen Übertragungselementen mit einer dem Spiraldurchmesser vergleichbaren Länge. Andererseits ist die Übertragungsqualität robust gegenüber einer Verdrehung der beiden Übertragungselemente 12a, 18a bzgl. einer gemeinsamen Zentralachse 50, so dass die betreffenden Kopplerelemente 8, 10 rotationssymmetrisch ausgestaltet werden können, was die Verbindung erleichtert.

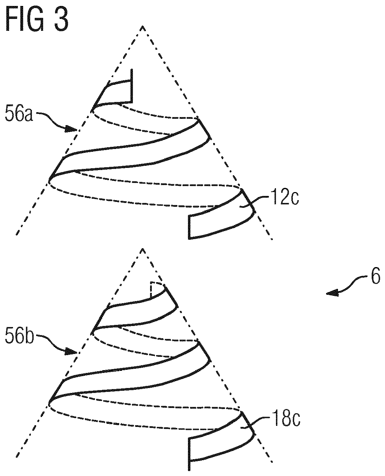

In FIG 3 ist in einer Seitenansicht eine weitere Ausgestaltungsform der Übertragungselemente 12c, 18c eines Kopplers 6 gezeigt. Vorliegend sind die beiden Übertragungselemente 12c, 18c lokal streifenförmig ausgebildet, und jeweils entlang von zueinander axial verschobenen Mantelflächen von Kegeln 56a, 56b mit gleichem Öffnungswinkel spiralförmig aufgewickelt. Die durch die Übertragungselemente 12c, 18c ausgebildeten Streifen verlaufen dabei jeweils entlang der Mantelflächen der Kegel 56a, 56b.

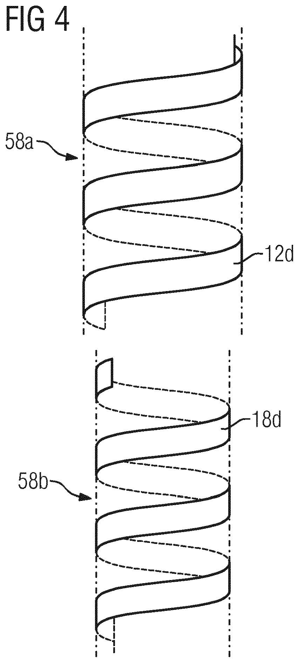

FIG 4 zeigt in einer Seitenansicht eine ähnliche Ausgestaltung, wobei das erste Übertragungselement 12d und das zweite Übertragungselement 18d jeweils lokal streifenförmig ausgebildet sind, und jeweils entlang von koaxialen Mantelflächen von Zylindern 58a, 58b aufgewickelt sind. Die durch die Übertragungselemente 12d, 18d ausgebildeten Streifen verlaufen dabei jeweils entlang der Mantelflächen der Zylinder 58a, 58b.

In den in FIG 3 und FIG 4 gezeigten Ausführungsformen lässt sich das betreffende erste Kopplerelement 8 jeweils als eine Art Hülse ausgestalten, in welche das zweite Kopplerelement 10 für die bestimmungsgemäße Signalübertragung bevorzugt formschlüssig einzuführen ist.
Obwohl die Erfindung im Detail durch das bevorzugte Ausführungsbeispiel näher illustriert und beschrieben wurde, ist die Erfindung nicht durch dieses Ausführungsbeispiel eingeschränkt. Andere Variationen können vom Fachmann hieraus abgeleitet werden, ohne den Schutzumfang der Erfindung zu verlassen.

## Patentansprüche

1. Koppler (6) zur kontaktlosen Signalübertragung bei hohen Datenraten, umfassend ein erstes Kopplerelement (8) mit einem ersten Übertragungselement(12, 12a-d) und ein zweites Kopplerelement (10) mit einem zweiten Übertragungselement(18, 18a-d),
wobei an einem ersten Ende (30) des ersten Übertragungselements (12, 12a-d) ein erster Signalkontakt (14) zur Verbindung mit einem Signalleiter (16) angeordnet ist,
wobei an einem ersten Ende (42) des zweiten Übertragungselements (18, 18a-d) ein zweiter Signalkontakt (20) zur Verbindung mit einem Signalleiter (4) angeordnet ist,
wobei das erste Übertragungselement (12, 12a-d) und das zweite Übertragungselement (18, 18a-d) jeweils entlang zueinander komplementären Rotationsflächen (56a, 56b, 58a, 58b) verlaufen,
wobei das erste Übertragungselement (12, 12a-d) zwischen dem ersten Ende (30) und einem zweiten (32) Ende eine Mehrzahl an ersten U-förmigen Kurven (37) ausbildet und dabei eine Spirale ausbildet oder um eine Mantelfläche eines Zylinders (58a) gewickelt ist,
wobei das zweite Übertragungselement (18, 18a-d) zwischen dem ersten Ende (42) und einem zweiten Ende (44) eine Mehrzahl an zweiten U-förmigen Kurven ausbildet und dabei eine Spirale ausbildet oder um eine Mantelfläche eines Zylinders (58b) gewickelt ist,
wobei das erste Übertragungselement (12, 12a-d) dazu eingerichtet ist, im Betrieb ein am ersten Signalkontakt (14) eingespeistes Signal mittels Richtkopplung an das zweite Übertragungselement (18, 18a-d) zu übertragen, und
wobei das zweite Übertragungselement (18, 18a-d) dazu eingerichtet ist, im Betrieb das Signal vom ersten Übertragungselement (12, 12a-d) zu empfangen und über den zweiten Signalkontakt (20) auszugeben.

2. Koppler (6) nach Anspruch 1,
wobei das erste Kopplerelement (8) rotationssymmetrisch ausgebildet ist, und das zweite Kopplerelement (10) komplementär zum ersten Kopplerelement (8) ausgebildet ist.

3. Koppler (6) nach einem der vorhergehenden Ansprüche,
wobei das erste Übertragungselement(12, 12a-d) und/oder das zweite Übertragungselement (18, 18a-d) wenigstens abschnittweise lokal flächenartig ausgebildet sind.

4. Koppler (6) nach einem der vorhergehenden Ansprüche,
wobei das erste Übertragungselement (12, 12a-d) auf einem dielektrischen Substrat (46) aufgebracht ist, welches eine Oberfläche des ersten Kopplerelements (8) bildet.

5. Koppler (6) nach einem der vorhergehenden Ansprüche,
wobei im ersten Kopplerelement (8) eine erste Spule (22) zur induktiven Energieübertragung angeordnet ist,
wobei im zweiten Kopplerelement (10) eine zweite Spule (24) zur induktiven Energieübertragung angeordnet ist, und
wobei die erste Spule (22) und die zweite Spule (24) dazu eingerichtet sind, im Betrieb des Kopplers (6) Energie vom ersten Kopplerelement (8) zum zweiten Kopplerelement (10) und/oder vom zweiten Kopplerelement (10) zum ersten Kopplerelement (8) zu übertragen.

6. Koppler (6) nach einem der vorhergehenden Ansprüche,
wobei das erste Kopplerelement (8) und das zweite Kopplerelement (10) jeweils einen elektromagnetischen Absorber (26, 28) aufweisen.

7. Koppler (6) nach einem der vorhergehenden Ansprüche, welcher für eine Übertragung von Signalen in einem Frequenzbereich von 200 MHz bis 4 GHz eingerichtet ist.

8. Magnetresonanztomograph (2) mit einer Lokalspule (1) und einem Koppler (6) nach einem der vorhergehenden Ansprüche,
wobei das erste Kopplerelement (8) des Kopplers (6) an der Lokalspule (1) angeordnet ist.

## Claims

1. Coupler (6) for contactless signal transmission with high data rates, comprising a first coupler element (8) with a first transmission element (12, 12a-d) and a second coupler element (10) with a second transmission element (18, 18a-d),
wherein a first signal contact (14) for connection to a signal conductor (16) is arranged on a first end (30) of the first transmission element (12, 12a-d),
wherein a second signal contact (20) for connection to a signal conductor (4) is arranged on a first end (42) of the second transmission element (18, 18a-d),
wherein the first transmission element (12, 12a-d) and the second transmission element (18, 18a-d) each extend along mutually complementary surfaces of revolution (56a, 56b, 58a, 58b),
wherein the first transmission element (12, 12a-d) forms a plurality of first U-shaped curves (37) between the first end (30) and a second (32) end and in doing so forms a spiral or is wound about a lateral surface of a cylinder (58a),
wherein the second transmission element (18, 18a-d) forms a plurality of second U-shaped curves between the first end (42) and a second end (44) and in doing so forms a spiral or is wound about a lateral surface of a cylinder (58b),
wherein the first transmission element (12, 12a-d) is configured, in operation, to transmit a signal supplied at the first signal contact (14) to the second transmission element (18, 18a-d) by means of directional coupling and
wherein the second transmission element (18, 18a-d) is configured, in operation, to receive the signal from the first transmission element (12, 12a-d) and output it via the second signal contact (20).

2. Coupler (6) according to claim 1,
wherein the first coupler element (8) is embodied as rotationally symmetrical and the second coupler element (10) is embodied as complementary to the first coupler element (8).

3. Coupler according to one of the preceding claims,
wherein the first transmission element (12, 12a-d) and/or the second transmission element (18, 18a-d), at least in sections, are embodied as locally flat.

4. Coupler according to one of the preceding claims,
wherein the first transmission element (12, 12a-d) is applied to a dielectric substrate (46) that forms a surface of the first coupler element (8).

5. Coupler according to one of the preceding claims,
wherein a first coil (22) for inductive energy transmission is arranged in the first coupler element (8),
wherein a second coil (24) for inductive energy transmission is arranged in the second coupler element (10) and
wherein the first coil (22) and the second coil (24) are configured, when the coupler (6) is in operation, to transmit energy from the first coupler element (8) to the second coupler element (10) and/or from the second coupler element (10) to the first coupler element (8).

6. Coupler according to one of the preceding claims,
wherein the first coupler element (8) and the second coupler element (10) each comprise an electromagnetic absorber (26, 28) .

7. Coupler according to one of the preceding claims,
which is configured for the transmission of signals in a frequency range of from 200 MHz to 4 GHz.

8. Magnetic resonance imaging system (2) with a local coil (1) and a coupler according to one of the preceding claims,
wherein the first coupler element (8) of the coupler (6) is arranged on the local coil (1).

## Revendications

1. Coupleur (6) de transmission sans contact du signal à de hauts taux de données, comprenant un premier élément (8) de coupleur, ayant un premier élément (12, 12a-d) de transmission et un deuxième élément (10) de coupleur ayant un deuxième élément (18, 18a-d) de transmission,
dans lequel, à une première extrémité (30) du premier élément (12, 12a-d) de transmission, est disposé un premier contact (14) de signal de liaison à un conducteur (16) de signal,
dans lequel, à une première extrémité (42) du deuxième élément (18, 18a-d) de transmission, est disposé un deuxième contact (20) de signal de liaison à un conducteur (4) de signal,
dans lequel le premier élément (12, 12a-d) de transmission et le deuxième élément (18, 18a-d) de transmission s'étendent chacun le long de surfaces (56a, 56b, 58a, 58b) de rotation complémentaires l'une de l'autre,
dans lequel le premier élément (12, 12a-d) de transmission constitue, entre la première extrémité (30) et une deuxième extrémité (32), une pluralité de premières courbes (37) en forme de U et constitue ainsi une spirale ou est enroulé autour d'une surface latérale d'un cylindre (58a),
dans lequel le deuxième élément (18, 18a-d) de transmission constitue, entre la première extrémité (42) et une deuxième extrémité (44) une pluralité de deuxièmes courbes en forme de U et constitue ainsi une spirale ou est enroulé autour d'une surface latérale d'un cylindre (58b),
dans lequel le premier élément (12, 12a-d) de transmission est conçu pour, en fonctionnement, transmettre au deuxième élément (18, 18a-d) de transmission, au moyen d'un couplage directionnel, un signal injecté au premier contact (14) de signal et
dans lequel le deuxième élément (18, 18a-d) de transmission est conçu pour, en fonctionnement, recevoir le signal du premier élément (12, 12a-d) de transmission et l'émettre par l'intermédiaire du deuxième contact (20) de signal.

2. Coupleur (6) suivant la revendication 1,
dans lequel le premier élément (8) de coupleur est de révolution et le deuxième élément (10) de coupleur est constitué de manière complémentaire du premier élément (8) de coupleur.

3. Coupleur (6) suivant l'une des revendications précédentes,
dans lequel le premier élément (12, 12a-d) de transmission et/ou le deuxième élément (18, 18a-d) de transmission sont constitués, au moins par endroit, du type suivant une surface localement.

4. Coupleur (6) suivant l'une des revendications précédentes,
dans lequel le premier élément (12, 12a-d) de transmission est déposé sur un substrat (46) diélectrique, qui forme une surface du premier élément (8) de coupleur.

5. Coupleur (6) suivant l'une des revendications précédentes,
dans lequel, dans le premier élément (8) de coupleur, est disposée une première bobine (22) de transmission d'énergie par induction,
dans lequel, dans le deuxième élément (10) de coupleur, est disposée une deuxième bobine (24) de transmission d'énergie par induction et
dans lequel la première bobine (22) et la deuxième bobine (24) sont conçues pour, lorsque le coupleur (6) fonctionne, transmettre de l'énergie du premier élément (8) de coupleur au deuxième élément (10) de coupleur et/ou du deuxième élément (10) de coupleur au premier élément (8) de coupleur.

6. Coupleur (6) suivant l'une des revendications précédentes,
dans lequel le premier élément (8) de coupleur et le deuxième élément (10) de coupleur ont chacun un absorbeur (26, 28) électromagnétique.

7. Coupleur (6) suivant l'une des revendications précédentes,
qui est conçu pour une transmission de signaux dans un domaine de fréquence de 200 MHz à 4 GHz.

8. Tomographe (2) à résonnance magnétique, comprenant une bobine (1) locale et un coupleur (6) suivant l'une des revendications précédentes, le premier élément (8) du coupleur (6) étant monté sur la bobine (1) locale.
